(19) Europäisches Patentamt

European Patent Office

Office européen des brevets

(11) Numéro de publication: **0 211 109**

A1

(12) **DEMANDE DE BREVET EUROPEEN**

(21) Numéro de dépôt: 85201267.3

(22) Date de dépôt: 05.08.85

(51) Int. Cl.⁴: **C12M 1/00** , C12M 1/04 , C12M 1/18

(43) Date de publication de la demande:
25.02.87 Bulletin 87/09

(84) Etats contractants désignés:
AT BE CH DE FR GB IT LI LU NL SE

(71) Demandeur: **ARBIOS Société Anonyme**
**Avenue Emile Rousseau**
**B-6000 Charleroi(BE)**

(72) Inventeur: **Desmons, Pierre**
**Rue L. Delvaux, 19**
**B-1400 Nivelles(BE)**
Inventeur: **Muller, Philippe**
**Rue d'Egypte**
**B-6312 Reves(BE)**

(74) Mandataire: **Bossard, Franz et al**
**ACEC - Service des Brevets Boîte Postale 4**
**B-6000 Charleroi(BE)**

(54) **Fermenteur à cuve à fonctionnement continu.**

(57) Un fermenteur à cuve est disposé de manière que l'axe de la cuve est approximativement horizontal, et comprend des parois pleines (3) perpendiculaires à l'axe, séparant la cuve en plusieurs chambres (4,5 6,7),les parois (3) étant munies de fenêtres (8) laissant passer le liquide disposées à des hauteurs déterminées; en outre à la partie haute des chambres des tuyaux d'évacuation de gaz sont prévus.

EP 0 211 109 A1

## FERMENTEUR A CUVE A FONCTIONNEMENT CONTINU.

La présente invention a pour objet un fermenteur à cuve dans lequel un liquide est soumis à l'action de micro-organismes fixés sur des supports solides ou formant des amas de cellules à mouvements libres obtenus par agglomération ou floculation et possédant des tailles suffisamment importantes pour être retenus par des grilles ou treillis appropriés. Les supports solides peuvent être soit à mouvement limité, tels que des lanières, fixées sur une grille, soit à mouvement libre, tels que granules ou billes en un matériau de densité plus grande que la densité du liquide, nageant librement dans le liquide. Les supports solides peuvent notamment être des billes en un gel approprié, par exemple alginates, acrylamides, etc. Dans ce fermenteur, le liquide circule en continu d'une tuyauterie d'entrée disposée à une de ses extrémités vers une tuyauterie de sortie à l'autre extrémité.

De tels fermenteurs sont en principe connus; ils sont constitués par exemple en une cuve cylindrique à axe vertical, la tuyauterie d'entrée étant disposée, suivant le cas soit en bas, soit en haut de la cuve et la tuyauterie de sortie à l'autre extrémité de la cuve.

Dans ces fermenteurs connus, les supports de micro-organismes, bien que de densité plus grande que le liquide sont souvent entraînés vers le haut à cause des gaz absorbés ou adsorbés ou captés à la surface ou emprisonnés dans des pores et forment des voûtes de très grande épaisseur, empêchant un fonctionnement normal du fermenteur. Les effets néfastes dûs à la formation de voûtes peuvent être réduits, si la cuve est séparée au moyen de treillis horizontaux en plusieurs chambres superposées. Même dans ce dernier cas, le tassement des supports contre les treillis peut sérieusement perturber un bon fonctionnement du fermenteur.

L'invention a pour but d'éviter ces inconvénients, de rendre la fermentation plus efficace, et de doter l'installation d'un rendement total plus grand par rapport au rendement d'une installation connue de même volume du fermenteur.

Pour atteindre ce but, le fermenteur suivant l'invention est constitué d'une cuve dont l'axe est disposé approximativement en direction horizontale ; en outre la cuve est séparée en plusieurs chambres au moyen de parois perpendiculaires à l'axe de la cuve ; les parois sont munies de fenêtres laissant passer le liquide en direction horizontale ; les fenêtres sont disposées à des hauteurs déterminées et à la partie haute des chambres sont prévus des tuyaux d'évacuation des gaz.

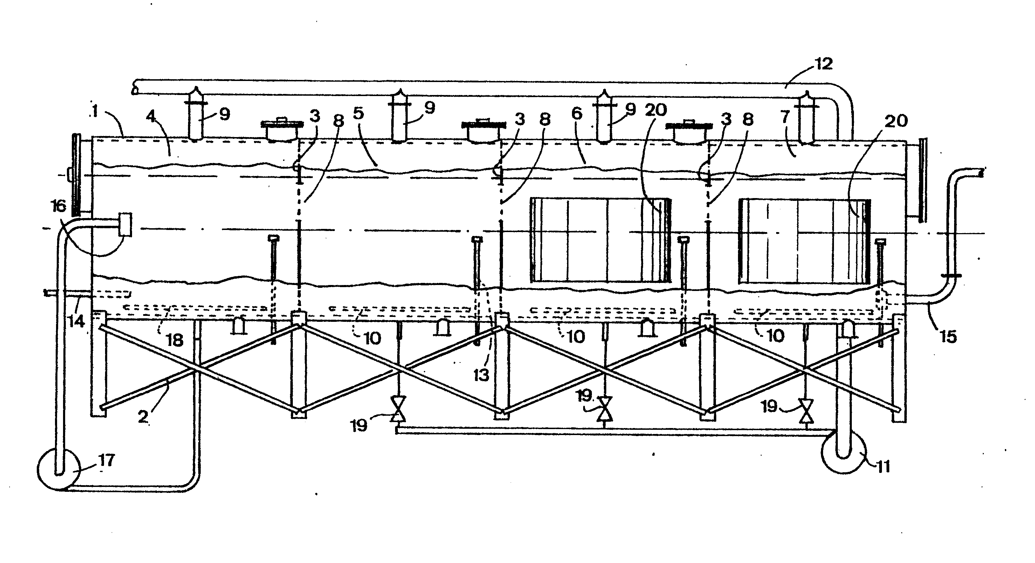

L'invention est expliquée ci-dessous à l'aide d'un exemple d'une forme d'éxécution en se référant au dessin annexé. La figure 1 du dessin représente une couple schématique à travers un fermenteur suivant l'invention.

A la figure 1, une cuve 1 est supportée par un bâti 2 de manière que l'axe de la cuve s'étende en direction horizontale. La section de la cuve peut être carrée, mais de préférence, elle est circulaire. Le diamètre d'une telle cuve peut être choisi en fonction de la fermentation envisagée. Dans le cas d'une fermentation transformant de la melasse est transformée en éthanol, une bonne grandeur est un diamètre entre 1 et 5 m.

La cuve 1 est séparée au moyen de parois 3 en plusieurs chambres 4,5,6,7 de longueur égale environ au diamètre de la cuve. D'une manière générale. de très bon résultats peuvent être obtenus lorsque la longueur des chambres est comprise entre un demi-diamètre et deux diamètres de la cuve. Toutefois, ces limites ne doivent pas être considérées comme restrictives. Les différentes chambres peuvent avoir des longueurs inégales.

Les parois 3 comprennent des fenêtres 8 à la hauteur environ du niveau du liquide dans la cuve. Ce niveau est maintenu de préférence entre environ 0,6 et 0,8 D, D étant le diamètre de la cuve. Lorsque les supports de micro-organismes peuvent exécuter des mouvements limités seulement (supports en forme de lameiles de densité plus faible que la densité du liquide dont une des extrémités est fixée au bas de la chambre, par exemple) les fenêtres sont ouvertes. Dans le cas où les amas ou supports de micro-organismes sont à mouvement libre, par exemple des billes en gel alginate de densité plus grande que celle du liquide, les fenêtres 8 sont obturées par des treillis en matière inerte par rapport au liquide à traiter aux supports et aux micro-organismes fixés ou non sur les supports (par exemple en acier inoxydable). Le terme treillis est utilisé ici pour désigner tout agencement quelconque assurant la fonction d'un treillis : laisser passer le liquide tout en interdisant le passage des amas ou supports de micro-organismes, grâce par exemple au choix des mailles du treillis. Les fenêtres 8, au lieu de se trouver à la hauteur du niveau du liquide, peuvent être disposées, dans ce cas, à un niveau beaucoup plus bas, notamment en un endroit où les supports de micro-organismes exécutent des mouvements parallèles aux parois 3 et ne manifestent pas ainsi de tendance de coller contre le treillis et d'en colmater les ouvertures.

A la partie haute de chaque chambre se trouve un tuyau d'évacuation 9 pour les gaz, tandis qu'au fond des chambres 5,6,7 est disposé un injecteur de gaz 10. Les injecteurs 10 sont reliés à la sortie d'un compresseur 11 dont l'entrée est reliée à un conduit 12 dans lequel débouchent les tuyaux 9. Des sondes de prise d'échantillons 13 peuvent être disposées dans chacune des chambres 4 à 7. Une tuyauterie d'entrée 14 amène le liquide à traiter dans la chambre 4. Le liquide passe ensuite par les fenêtres 8 de chambre en chambre et quitte finalement la chambre 7 par une tuyauterie de sortie 15. Le niveau du liquide à l'intérieur des chambres 4,5,6,7 est maintenu à une hauteur désirée par exemple grâce à une disposition appropriée de la tuyauterie de sortie (exemple : col de cygne comme montré à la figure 1).

Si par exemple, dans la première chambre 4, la fermentation est très active et peut donner lieu à l'apparition de mousse, un injecteur de gaz peut ne pas être prévu au fond de la chambre 4. Si cependant un effet de brassage est souhaitable dans cette première chambre, il est préférable de produire cet effet au moyen d'une injection de liquide, éventuellement additionné d'un agent anti-mousse. Dans ce but, le liquide alimentaire peut être injecté avec une certaine pression et dans une direction désirée. Par exemple grâce à une disposition appropriée de la tuyauterie d'amenée, non représentée, et la mise sous pression du liquide alimentaire.

Suivant un autre variante, montrée à la figure 1, un prélèvement de liquide est prévu à l'endroit d'un orifice 16 relié à une pompe 17. La sortie de la pompe alimente ensuite une tuyère d'injection 18 au bas de la chambre 4. Il est possible, et parfois souhaitable, que la deuxième chambre 5 soit équipée également d'un injecteur de liquide qui peut être alimenté par la même pompe 17.

Le prélèvement de liquide peut être opéré à travers un treillis qui retient les supports ou amas de micro-organismes et laisse seulement passer le liquide ou au contraire peut englober aussi le prélèvement de supports de micro-organismes qui sont ensuite réinjectés simultanément avec le liquide.

Le volume apparent des supports pour les micro-organismes dans chaque chambre peut être différent dans chaque chambre et la nature des supports et les micro-organismes sur chaque sorte de support peuvent aussi être différents dans chaque chambre. En particulier, les micro-organismes dans chaque chambre peuvent être choisis en fonction des conditions de reproduction et de longévité favorables propres à la composition du liquide dans chacune des chambres. Cependant, comme les conditions dans les différentes chambres ne varient pas dans des proportions très importantes, en général, il est possible et plus simple d'équiper toutes les chambres de manière identique.

Il convient d'observer ici, que la cuve du fermenteur suivant l'invention est séparée en chambres quasi indépendantes. La cuve en son entièreté n'est donc pas un fermenteur infiniment mélangé. Par contre, chaque chambre se présente individuellement comme un fermenteur infiniment mélangé, mais différent des fermenteurs des chambres adjacentes. Cela est vrai aussi si les mêmes supports de mêmes micro-organismes sont utilisés en mêmes concentrations dans toutes les chambres.

Dans chaque chambre, des mesures appropriées peuvent être prises pour assurer un brassage convenable ou pour porter à son optimum le rendement des réactions biologiques. Ainsi, si la réaction biologique est favorisée par une petite quantité d'air ou un autre adjuvant gazeux, le compresseur 1 peut présenter une entrée réglable pour l'air ou l'adjuvant gazeux qui est ensuite mélangé en faible proportion aux gaz récoltés par les tuyaux 9 et envoyé dans les différentes chambres en quantités déterminées, par exemple en quantité relativement faible dans la chambre 5 et en quantités croissantes dans les chambres suivantes où l'activité de fermentation est de plus en plus faible. Le réglage de ces quantités se fait par exemple au moyen de vannes 19 ou au moyen d'autres accessoires appropriés, éventuellement commandés par une commande centrale gouvernée par ordinateur en fonction de paramètres et de grandeurs mesurées, convenablement choisies.

Lorsque le liquide est de la mélasse en solution aqueuse et les micro-organismes sont des levures (par exemple Saccharomyces species) ou des bactéries (par exemple Zymomonas species) la concentration du liquide en sucres à l'entrée 14 peut être très importante, supérieure, par exemple, à 15%. A la sortie 15, cette concentration peut tomber à une valeur inférieure à 1% si le temps de séjour du liquide dans le fermenteur atteint au moins 4 heures. Lors de cette fermentation, jusqu'à 95% du sucre sont transformés en éthanol et anhydride d'acide carbonique. La production d'éthanol peut atteindre ainsi un ordre de grandeur de 25 kg par heure et mètre cube d'influent.

Notamment dans le cas de fermenteurs de grande taille, il peut être avantageux de prendre des mesures pour assurer un excellent mélange entre liquide et amas ou supports de micro-organismes. De telles mesures sont par exemple la disposition en face des injecteurs 10 ou 18 de tuyaux médians 20 séparant des zones de mouvements opposés du liquide dans les chambres, par exemple des zones à mouvement ascendant et zones à mouvement descendant.

Lorsque la fermentation est très intense, le refroidissement naturel à travers les parois de la cuve peut être insuffisant. Dans ce cas, des moyens de refroidissement du liquide doivent être prévus. Pour des cuves de capacité relativement faible une enceinte refroidie de la cuve, par exemple à double paroi peut suffire. Pour des capacités plus grandes, il est possible de disposer un faisceau de tubes de refroidissement en un endroit approprié de chaque chambre que nécessite un refroidissement intense. Les tubes de refroidissement peuvent notamment se trouver dans l'espace entourant le tuyau 20. Il est possible aussi de refroidir toutes les chambres au moyen de tuyaux droits traversant la cuve 1 de part en part. Dans ce dernier cas les tuyaux peuvent être disposés par exemple au dessus ou en dessous des tuyaux 20 ou placés le long de la paroi cylindrique de la cuve 1 à des distances telles de celle-ci que, de préférence, mais non nécessairement, elles ne traversent pas les tuyaux 20.

Par contre, il est notamment possible de fixer les tuyaux 20 à ces tubes de refroidissement et de profiter ainsi de la bonne conductibilité calorifique du matériau des tuyaux 20.

**Revendications**

1° Fermenteur à cuve dans lequel un liquide est soumis à l'action de micro-organismes formant des amas de cellules ou fixés sur des supports solides à mouvement limité ou à mouvement libre et dans lequel le liquide circule en continu d'une tuyauterie d'entrée vers une tuyauterie de sortie,

caractérisé en ce que le fermenteur est constitué par une cuve (1) dont l'axe est disposé approximativement en direction horizontale, en ce que la cuve est séparée en plusieurs chambres (4,5,6,7) au moyen de parois pleines (3) perpendiculaires à l'axe de la cuve, en ce que les parois (3) sont munies de fenêtres (8) laissant passer le liquide disposées à des hauteurs déterminées, et en ce qu'à la partie haute des chambres des tuyaux d'évacuation de gaz sont prévus.

2° Fermenteur suivant la revendication 1, caractérisé en ce que les fenêtres (8) sont obturées par des treillis

3° Fermenteur suivant une des revendications 1 et 2, caractérisé en ce qu'au moins dans une partie des chambres des injecteurs de gaz (10) ou de liquide (18) sont disposés au fond des chambres

4° Fermenteur suivant la revendication 3, caractérisé en ce qu'un compresseur (11) récolte une partie des gaz récoltés par les tuyaux d'évacuation de gaz (9) et les réinjecte dans des injecteurs de gaz (10)

5° Fermenteur suivant la revendication 4, caractérisé en ce que le compresseur comprend une prise d'entrée d'un adjuvant gazeux favorisant la fermentation

6° Fermenteur suivant la revendication 3, caractérisé en ce qu'une pompe (17) renvoie du liquide aspiré par une ouverture (16) dans un ou plusieurs injecteurs de liquide (18)

7° Fermenteur suivant une des revendications précédentes, caractérisé en ce qu'au moins une chambre (4,5,6,7) comprend une prise d'échantillon (13)

8° Fermenteur suivant une des revendications précédentes, caractérisé en ce que le volume apparent des supports de micro-organismes représente plus que le tiers du volume du liquide dans chaque chambre

9° Fermenteur suivant une des revendications précédentes, caractérisé en ce qu'il comprend des moyens de refroidissement du liquide.

10° Fermenteur suivant une des revendications précédentes, caractérisé en ce qu'au moins dans une des chambres est disposé un tuyau médian séparant des zones de mouvements opposés du liquide dans la chambre

11° Fermenteur suivant une des revendications précédentes, caractérisé en ce que le niveau du liquide dans les chambres se trouve à une hauteur comprise entre 0,6 et 0,8D, D étant le diamètre de la cuve.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Ci⁴) |
|---|---|---|---|
| A | FR-A- 680 303 (E. STICH)<br>* Résumé; figure 1; page 2, ligne 61 - page 3, ligne 14 * | 1-11 | C 12 M 1/00<br>C 12 M 1/04<br>C 12 M 1/18 |
| | --- | | |
| A | EP-A-0 070 245 (M. GUERIN)<br>* Revendication 1; figure 1 * | 1 | |
| | --- | | |
| A | EP-A-0 102 279 (CHAMBRE D'AGRICULTURE DE L'AISNE)<br>* Revendication 1 * | 1 | |
| | ----- | | |

DOMAINES TECHNIQUES RECHERCHES (Int. Cl.4)

C 12 M

Le présent rapport de recherche a été etabli pour toutes les revendications

| Lieu de la recherche | Date d'achèvement de la recherche | Examinateur |
|---|---|---|
| LA HAYE | 04-03-1986 | VAN MOER A.M.J. |